# EUROPÄISCHE PATENTSCHRIFT

(11) **EP 1 490 518 B1**
(45) Veröffentlichungstag und Bekanntmachung des Hinweises auf die Patenterteilung: **25.06.2014**
(21) Anmeldenummer: 03744778.6
(22) Anmeldetag: 06.02.2003
(51) Int. Cl.: C12Q 1/68

(54) **VERFAHREN ZUM NACHWEIS UND ZUR DIFFERENZIERUNG DES MYCOBACTERIUM TUBERCULOSIS-KOMPLEX**
METHOD FOR DETECTION AND DIFFERENTIATION OF MYCOBACTERIUM TUBERCULOSIS COMPLEX
PROCEDE DE DETECTION ET DE DIFFERENCIATION DU COMPLEXE MYCOBACTERIUM TUBERCULOSIS

(30) Priorität: 26.03.2002 DE 10213537
(43) Veröffentlichungstag der Anmeldung: 29.12.2004
(73) Patentinhaber: Hain Lifescience GmbH, 72147 Nehren (DE)
(72) Erfinder: WEIZENEGGER, Michael, 69168 Wiesloch (DE)
(74) Vertreter: Keller, Günter
(86) Internationale Anmeldenummer: PCT/EP2003/001197
(87) Internationale Veröffentlichungsnummer: WO 2003/080871

(56) Entgegenhaltungen:
- EP-A- 1 098 003
- WO-A-01/66797
- WO-A-02/22872
- WO-A-02/097126
- US-A- 5 703 217
- US-A- 5 736 365
- DATABASE EMBL [Online] 5. März 2001 (2001-03-05) "Mycobacterium tuberculosis open reading frame mtbn1" retrieved from EBI Database accession no. AAA89035 XP002263240
- DATABASE EMBL [Online] 28. März 2000 (2000-03-28) NIEMANN S.: "Mycobacterium bovis subsp. caprae partial gyrB gene for DNA gyrase subunit B" retrieved from EBI Database accession no. AJ276122 XP002263241
- DATABASE EMBL [Online] 24. Januar 2000 (2000-01-24) KASAI H.: "Mycobacterium microti gyrB gene for DNA gyrase B subunit, partial cds, strain T 901" retrieved from EBI Database accession no. AB014205 XP002263242
- NIEMANN S. ET AL.: "Differentiation of clinical Mycobacterium tuberculosis complex isolates by gyrB DNA sequence polymorphism analysis" JOURNAL OF CLINICAL MICROBIOLOGY, Bd. 38, Nr. 9, September 2000 (2000-09), Seiten 3231-3234, XP002263235 ISSN: 0095-1137 in der Anmeldung erwähnt
- KASAI HIROAKI ET AL: "Differentiation of phylogenetically related slowly growing mycobacteria by their gyrB sequences" JOURNAL OF CLINICAL MICROBIOLOGY, Bd. 38, Nr. 1, Januar 2000 (2000-01), Seiten 301-308, XP002263236 ISSN: 0095-1137 in der Anmeldung erwähnt
- TALBOT E.A. ET AL.: "PCR identification of Mycobacterium bovis BCG" JOURNAL OF CLINICAL MICROBIOLOGY, Bd. 35, Nr. 3, 1997, Seiten 566-569, XP002263237 ISSN: 0095-1137 in der Anmeldung erwähnt
- BODEN D. ET AL.: "Reverse hybridization assay for rapid identification of mycobacteria from cultured samples" CLINICAL LABORATORY, Bd. 44, Nr. 9, September 1998 (1998-09), Seiten 687-692, XP009021969
- LIESACK W. ET AL.: "COMPLETE NUCLEOTIDE SEQUENCE OF THE MYCOBACTERIUM LEPRAE 23 S AND 5S RRNA GENES PLUS FLANKING REGIONS AND THEIR POTENTIAL IN DESIGNINGDIAGNOSTIC OLIGONUCLEOTIDE PROBES" FEBS LETTERS, Bd. 281, Nr. 1/2, April 1991 (1991-04), Seiten 114-118, XP000984427 ISSN: 0014-5793
- TORTOLI E. ET AL.: "Performance assessment of new multiplex probe assay for identification of Mycobacteria" JOURNAL OF CLINICAL MICROBIOLOGY, Bd. 39, Nr. 3, März 2001 (2001-03), Seiten 1079-1084, XP002263238 ISSN: 0095-1137
- FU L.M. ET AL.: "IS MYCOBACTERIUM TUBERCULOSIS A CLOSER RELATIVE TO GRAM-POSITIVE OR GRAM-NEGATIVE BACTERIAL PATHOGENS?" TUBERCULOSIS, Bd. 82, Nr. 2/3, 2002, Seiten 85-90, XP009002056 ISSN: 1472-9792
- PARSONS L.M. ET AL.: "Rapid and simple approach for identification of Mycobacterium tuberculosis complex isolates by PCR-based genomic deletion analysis." JOURNAL OF CLINICAL MICROBIOLOGY. UNITED STATES JUL 2002, Bd. 40, Nr. 7, Juli 2002 (2002-07), Seiten 2339-2345, XP002263239 ISSN: 0095-1137
- RICHTER E. ET AL.: "EVALUATION OF GENOTYPE MTBC ASSAY FOR DIFFERENTIATION OF CLINICAL MYCOBACTERIUM TUBERCULOSIS COMPLEX ISOLATES" JOURNAL OF CLINICAL MICROBIOLOGY, Bd. 41, Nr. 6, Juni 2003 (2003-06), Seiten 2672-2675, XP008020272 ISSN: 0095-1137

### SEQUENCE LISTING

<110> Hain Lifescience GmbH
<120> Verfahren und Differenzierung des Mycobacterium tuberculosis-Komplex
<130> H30142
<160> 22
<170> PatentIn version 3.1
<210> 1
   <211> 25
   <212> DNA
   <213> mycobacterium tuberculosis complex
<400> 1
   tgacgacggc gcagctacgc tcgcg 25
<210> 2
   <211> 20
   <212> DNA
   <213> mycobacterium tuberculosis complex
<400> 2
   ggctcgaagt cgagatcaag 20
<210> 3
   <211> 20
   <212> DNA
   <213> mycobacterium tuberculosis complex
<400> 3
   ctcaccggtg acgatatccg 20
<210> 4
   <211> 18
   <212> DNA
   <213> mycobacterium tuberculosis complex
<400> 4
   ggacctaagg cgaggccg 18
<210> 5
   <211> 20
   <212> DNA
   <213> mycobacterium tuberculosis complex
<400> 5
   ggagggcagt aggggatgag 20
<210> 6
   <211> 19
   <212> DNA
   <213> mycobacterium tuberculosis complex
<400> 6
   cgggcggctg ggtgatgtg 19
<210> 7
   <211> 18
   <212> DNA
   <213> mycobacterium tuberculosis complex
<400> 7
   gccttcctgc gtcacccc 18
<210> 8
   <211> 21
   <212> DNA
   <213> mycobacterium tuberculosis complex
<400> 8
   attccgtggt ttcgaaaaca g 21
<210> 9
   <211> 21
   <212> DNA
   <213> mycobacterium tuberculosis complex
<400> 9
   gacacagcct tgttcacaac g 21
<210> 10
   <211> 14
   <212> DNA
   <213> mycobacterium tuberculosis complex
<400> 10
   cgtggtggag cgga 14
<210> 11
   <211> 16
   <212> DNA
   <213> mycobacterium tuberculosis complex
<400> 11
   acgggtacga gtggtc 16
<210> 12
   <211> 16
   <212> DNA
   <213> mycobacterium tuberculosis complex
<400> 12
   acgggtatga gtggtc 16
<210> 13
   <211> 16
   <212> DNA
   <213> mycobacterium tuberculosis complex
<400> 13
   cagaaccgca ccgttg 16
<210> 14
   <211> 16
   <212> DNA
   <213> mycobacterium tuberculosis complex
<400> 14
   cagaaccgta ccgttg 16
<210> 15
   <211> 17
   <212> DNA
   <213> mycobacterium tuberculosis complex
<400> 15
   ctggccgctg tgatctc 17
<210> 16
   <211> 17
   <212> DNA
   <213> mycobacterium tuberculosis complex
<400> 16
   ctggccgcgg tgatctc 17
<210> 17
   <211> 17
   <212> DNA
   <213> mycobacterium tuberculosis complex
<400> 17
   gtctgtaacg aacagct 17
<210> 18
   <211> 17
   <212> DNA
   <213> mycobacterium tuberculosis complex
<400> 18
   gtctgtaatg aacagct 17
<210> 19
   <211> 16
   <212> DNA
   <213> mycobacterium tuberculosis complex
<400> 19
   gactttcgcg tcggtg 16
<210> 20
   <211> 16
   <212> DNA
   <213> mycobacterium tuberculosis complex
<400> 20
   gactttcgag tcggtg 16
<210> 21
   <211> 15
   <212> DNA
   <213> mycobacterium tuberculosis complex
<400> 21
   ggagttctgg ggctg 15
<210> 22
   <211> 16
   <212> DNA
   <213> mycobacterium tuberculosis complex
<400> 22
   cgactacgcc tgtcgg 16

## Beschreibung

Die vorliegende Erfindung betrifft das Gebiet der Diagnostik von Mikroorganismen, insbesondere den Nachweis und die Differenzierung von Mykobakterien, die mit der Erkrankung der Tuberkulose assoziiert sind.

Der Nachweis von Bakterien und ihre genaue Identifizierung spielten eine sehr wichtige Rolle in der klinischen Diagnostik. So sollte ein bestimmter Erreger bei bakteriellen Infektionen möglichst schnell identifiziert werden, damit eine entsprechende Behandlung eingeleitet werden kann.

Ein Beispiel bakterieller Erreger sind Gram-positive Bakterien, insbesondere Mykobakterien. Gegenwärtig sind über 100 Mykobakterienspezies bekannt, die aus klinischem Material nachgewiesen und differenziert werden. Von besonderer Bedeutung sind hierbei die hochpathogenen Mykobakterienspezies des *Mycobacterium tuberculosis-Komplex,*

Der *Mycobacterium tuberculosis*(MTB)-Komplex wird durch die vier sehr eng verwandten Gruppen *M. tuberculosis, M. bovis, M. bovis* BCG, *M. africanum,* und *M. microti* definiert. Die enge Verwandtschaft dieser Spezies wurde durch DNS-DNS-Hybridisierungen (Baess, I. et al., 1979), Isoenzym Analysen (Feizabadi, M. et al., 1996) und Elektrokaryotypisierung (Feizabadi, M. et al., 1996) gezeigt. Jedoch sind Vorkommen und die Pathogenität dieser Spezies sehr unterschiedlich. *M. tuberculosis* und *M*. *africanum* sind nur im Menschen, *M. bovis* in einer Reihe von domestizierten und wilden Säugetieren und im Menschen zu finden. *M. bovis* BCG ist ein in seiner Pathogenität abgeschwächter *M. bovis-*Stamm. Er wird als Vaccinie gegen eine Infektion mit Spezies des MTB-Komplexes eingesetzt. BCG kann jedoch in Menschen mit geschwächter oder fehlender Immunkompetenz eine Infektion hervorrufen. Darum ist eine schnelle und sichere Diagnostik für BCG von großer Bedeutung. *M. microti* kann, wie kürzlich erst entdeckt wurde, nicht nur Wühlmäuse sondern auch Menschen infizieren.

In der medizinischen Routinediagnostik sowie in der Lebensmittelanalytik erfolgte die klassische Identifizierung von Bakterien über Selektivmedien und anschließende Untersuchung der biochemischen Eigenschaften. Dabei lässt sich aber oftmals nicht die genaue Spezies bestimmen. Zudem sind diese Untersuchungen sehr zeitaufwendig, und es muss für die weitergehenden Untersuchungen eine Reinkultur vorliegen.

In den letzten Jahren sind wichtige Erfindungen gemacht worden, um mit sehr speziesspezifische Primern in einer Nukleinsäureamplifikationsreaktion Organismen nachzuweisen. Die Detektion erfolgt dabei meist über Gelelektrophorese oder analog der ELISA-Technik über immobilisierte Sonden in Mikrotiterplatten. Gerade Bakteriengruppen von hoher Komplexität, großer Diversität und schwierigen Wachstumsbedingungen (z.B. sehr langsam wachsende Bakterien) sind der klassischen Kulturdifferenzierung schwer zugänglich und/oder verzögern die Diagnostik erheblich. Revolutionierend sind hier nukleinsäurebasierende Verfahren, die sich durch hohe Spezifität und Sensitivität auszeichnen.

Hierzu ist es notwendig, Amplifikationsprimer zu entwickeln, die konserviert in der Bakteriengruppe vorkommen, die es nachzuweisen und zu differenzieren gilt. Standardzielsequenz ist hier die ribosomale RNS (rRNS) oder die ribosomale DNS (rDNS) inklusive ihren Spacerstrukturen. Sie besitzt als ubiquitäres Molekül mit ihren konservativen und variablen Abschnitten sehr gute Eigenschaften für diese Fragestellungen. Die diagnostisch am häufigsten verwendete Zielsequenz ist die 16S rRNS. Sie ist in den entsprechenden Datenbanken am besten repräsentiert. Es sind immer wieder Anstrengungen unternommen worden, Spezies der sogenannten "langsam wachsenden" Mykobakterien, zu denen der MTB-Komplex gehört mit nukleinbasierenden Verfahren zu differenzieren. Alle diese Verfahren aber waren nicht in der Lage, Spezies des MTB-Komplex zu unterscheiden.

Kasai H. et al. (2000), (Differentiation of Phylogenetically Related Slowly Growing Mycobacteria by their gyrB Sequences. J.Clin.Microbiol. 38: 301-8 und Niemann S. et al. (2000) Differentiation of Clinical Mycobacterium tuberculosis Complex Isolates by gyrB DNA Sequence Polymorphism Analysis. J.Clin.Microbiol. 38: 3231-4) beschrieben DNS-Polymorphismen zur Unterscheidung einzelner Spezies des MTB-Komplex. Sie entwickelten zudem einen Analysemethode zur Differenzierung des MTB-Komplexes mit Hilfe der PCR und einem nachgeschalteten Restriktionsverdau [Restriktionslängenpolymorphismus(RFLP)].

Diese Methode ist aber nur in Forschungslaboratorien oder Einrichtungen mit molekulargenetisch erfahrenen Mitarbeitern durchzuführen. Der Auftrennung der DNS-Fragmente im Agarosegel folgt eine Färbereaktion mit hochmutagenen Farbstoffen (z.B. Ethidiumbromid). Zudem kann über das gyrB-Gen *M. bovis* nicht von *M. bovis* BCG unterschieden werden. Talbot E.A. et al. (1997) PCR Identification of M. bovis BCG. J.Clin.Microbiol. 35: 566-9 veröffentlichte einen PCR basierenden Essay zur Differenzierung von *M. bovis* und *M. bovis* BCG über eine große Deletion im RD1 Gen. Dieser Genpolymorphismus vermag wiederum nicht die anderen Mitglieder des MTB-Komplexes zu erkennen.

Die EP 1 098 003 beschreibt ein Verfahren zur Identifizierung von langsam wachsenden Mycobakterien, wobei charakteristische Nukleotidsequenzen verwendet werden, die in dem gyrB-Gen vorhanden sind.

In der nicht vorveröffentlichten WO 02/097126 werden Sequenzen offenbart, die mit den SEQ ID NO.: 4, 7 und 21 übereinstimmen.

Die WO 02/22872 offenbart ein Mulitplex PCR Verfahren zum Nachweis und zur Identifikation von Mykobakterien wobei Stämme von *M. tuberculosis* und *non-tuberculosis Mycobacteria* (NTM) identifiziert werden können.

Die WO 01/66797 offenbart ein Multiplex Hybridisierungsverfahren zur Identifikation von Pathogenenen Mykobakterien.

Die hier vorliegende Erfindung beschreibt besonders geeignete, neue Primer und Gensonden, die eine Differenzierung und einen Nachweis von Mykobakterien einschließlich der Differenzierung des MTB-Komplexes sehr spezifisch und hochsensitiv ermöglichen. Des weiteren ist Aufgabe der vorliegenden Erfindung, einen Test zur Differenzierung und zum Nachweis von Mykobakterien zur Verfügung zu stellen, der relativ einfach und schnell durchzuführen ist. Ein wesentlicher Teil der Erfindung ist das Multiplexverfahren zum simultanen Nachweis und zur Differenzierung des MTB-Komplexes unter Einbeziehung unterschiedlicher Genomstrukturen.

Diese Aufgabe wurde erfindungsgemäß gelöst durch ein Verfahren zum Nachweis und zur Differenzierung des *Mycobacterium tuberculosis-Komplex* gemäß Anspruch 1, bei welchem eine nachzuweisende Nukleinsäure, welche ein Fragment aus dem Genom eines *Mycobacterium tuberculosis-Komplex* oder komplementär zu diesem ist, an eine sequenz- und/oder speziesspezifische Nukleinsäuresonde unter stringenten Bedingungen hybridisiert und anschließend die nachzuweisende Nukleinsäure beziehungsweise die Hybridisierung der nachzuweisenden Nukleinsäure an die sequenzspezifische Nukleinsäuresonde detektiert wird, wobei die sequenzspezifische Nukleinsäuresonde ausgewählt ist aus den Sequenzen mit den SEQ ID No.: 10 - 20, 22 beziehungsweise komplementär zu diesen Sequenzen ist.

Diese Ausführungsform der Erfindung wird im folgenden kurz Hybridisierungsverfahren genannt. Der Begriff "Hybridisierungsverfahren" schließt alle bevorzugten Ausführungsformen ein.

Der Begriff "Nukleinsäure" und "Oligonukleotid" bezieht sich im Sinne der vorliegenden Erfindung auf Primer, Proben, Sonden und Oligomerfragmente, welche detektiert werden. Der Begriff Nukleinsäure und Oligonukleotid ist weiterhin generisch zu Polydesoxyribonukleotiden (enthaltend 2-Deoxy-D-Ribose) und zu Polyribonukleotiden (enthaltend D-Ribose) oder zu jedem weiteren Typ von Polynukleotid, das ein N-Glykosid einer Purinbase oder einer Pyrimidinbase ist, beziehungsweise einer modifizierten Purinbase oder einer modifizierten Pyrimidinbase. Eingeschlossen sind erfindungsgemäß auch PNA's, d. h. Polyamide mit Purin-/Pyrimidin-Basen. Die Begriffe Nukleinsäure und Oligonukleotid werden im Sinne der vorliegenden Erfindung nicht als verschieden angesehen, insbesondere soll die Verwendung der Begriffe keine Unterscheidung in Bezug auf die Länge bedeuten. Diese Begriffe schließen sowohl doppel- beziehungsweise einzelsträngige DNA, als auch doppel- beziehungsweise einzelsträngige RNA ein.

Erfindungsgemäß wird eine Zusammensetzung, welche die nachzuweisende Nukleinsäure oder einen Teil davon enthält, mit einer oder mit mehreren Sonden hybridisiert.

Prinzipiell ist es möglich, durch Hybridisierung mit einer einzelnen spezifischen Sonde die nachzuweisende Nukleinsäure und damit beispielsweise die Bakterienspezies zu bestimmen. Es ist aber auch möglich, die Zusammensetzung, welche die nachzuweisende Nukleinsäure oder einen Teil davon enthält, mit mehr als einer Sonde zu hybridisieren. Dadurch wird die Aussagekraft des Verfahrens erhöht. Man erhält dann ein genaues Profil und kann die nachzuweisende Nukleinsäure und damit beispielsweise die Bakterienspezies mit hoher Sicherheit bestimmen. Die letztgenannte Ausführungsform, d.h. die Hybridisierung der Zusammensetzung, welche die nachzuweisende Nukleinsäure oder einen Teil davon enthält, mit mehr als einer Sonde ist gemäß der vorliegenden Erfindung bevorzugt ob der besonderen praktischen Bedeutung. Um eine Hybridisierung mehrerer Sonden gleichzeitig zu ermöglichen, ist eine besondere Abstimmung der Sonden untereinander notwendig.

Daher ist ein Verfahren für die Differenzierung des M. Tuberculosis-Komplex (MTBK) ein Multiplexverfahren, bei dem die für die Differenzierung notwendigen bakteriellen Genomabschnitte in einer gemeinsamen Amplifikations- und Hybridisierungsreaktion angereichert und hybridisiert werden. Für diese Anwendung ist die Amplifikation verschiedener Genfragmente notwendig.

Im folgenden wird eine ausführliche Variante des Multiplexverfahren beschrieben. Die Sonden und Primer zur Detektion des MTBK liegen bevorzugt auf dem 23S ribosomalen RNS-Gen, die Sonden und Primer zur Unterscheidung der Spezies *M. tuberculosis, M. africanum, M. mikroti, M. bovis* und *M. bovis caprae* hybridisieren mit Abschnitten des Gyrasegens. Mit dieser Zielsequenz aber kann *M. bovis* nicht von BCG unterschieden werden. Dazu muss die Deletion des RD1-Gens von *M. bovis* herangezogen werden. Dies erfordert einen weiteren Primer und eine weitere Sonde. Da die zur Differenzierung notwendigen Polymorphismen auf dem Gyrasegen ca. 1000 Basen umfassen, ist zudem eine Aufspaltung oder -teilung in kleinere Fragmente für eine effektive Amplifikation wichtig. Die Vielzahl von Primern, die alle miteinander agieren können, erfordert ein sorgfältiges Design.

Ein wichtiger Teil des Verfahrens ist daher das Multiplex-Amplifikationsverfahren, bestehend aus 8 Primern und das "multigeplexte" reverse Hybridisierungsverfahren. Da hier Sonden mit unterschiedlicher Primärstruktur unter gleichen Bedingungen hybridisieren und teilweise Einzelbasenaustasche unterschieden werden müssen, sind bei der Stringenzwaschung Salze wie Betain oder Tetramethylammoniumchloid als Modulatoren der Waserstoffbrückenbindungen bevorzugt.

Das Multiplexamplifikationsverfahren wird bevorzugt mit den Primern gemäß SEQ ID No. 1-9 durchgeführt.

Die erfindungsgemäßen Nukleinsäuresonden, insbesondere die Nukleinsäuresonden mit den SEQ ID No.s 10-22, ermöglichen das multigeplexte reverse Hybridisierungsverfahren.

Dem Fachmann ist bewußt, daß ausgehend von der Lehre der vorliegenden Erfindung auch Sonden entworfen werden können, die geringfügig von den erfindungsgemäßen Sonden abweichen, aber dennoch funktionieren. So sind auch Sonden denkbar, die gegenüber den erfindungsgemäßen Sonden mit den Sequenzen SEQ ID No. 1-22 am 5'-und/oder 3'-Ende Verlängerungen oder Verkürzungen um wenigstens ein, zwei oder drei Nukleotide aufweisen. Ebenso ist denkbar, daß einzelne oder wenige Nukleotide einer Sonde durch andere Nukleotide austauschbar sind, solange die Spezifität der Sonde und der Schmelzpunkt der Sonde nicht zu stark verändert werden. Das schließt ein, daß bei Abwandlung die Schmelztemperatur der abgewandelten Sonde nicht zu stark von der Schmelztemperatur der ursprünglichen Sonde abweicht. Die Schmelztemperatur wird dabei nach der G (=4°C) + C ( =2°C) Regel ermittelt. Dem Fachmann ist klar, daß neben den üblichen Nukleotiden A, G, C, T (U) auch modifizierte Nukleotide, wie Inosin, 7-Deaza-2'-deoxy-guanosin usw. zur Anwendung kommen können. Die Lehre der vorliegenden Erfindung ermöglicht solche Modifikationen, ausgehend vom Gegenstand der Ansprüche.

Der Begriff "Hybridisierung" bezieht sich auf die Bildung von Duplexstrukturen durch zwei einzelsträngige Nukleinsäuren aufgrund von komplementärer Basenpaarung. Hybridisierung kann zwischen komplementären Nukleinsäuresträngen oder zwischen Nukleinsäuresträngen erfolgen, welche kleinere Regionen an Fehlpaarung aufweisen. Die Stabilität des Nukleinsäuren-Duplexes wird gemessen durch die Schmelztemperatur Tₘ. Die Schmelztemperatur Tₘ ist die Temperatur (unter definierter Ionenstärke und pH), bei welcher 50% der Basenpaare dissoziiert sind.

Bedingungen, bei denen lediglich vollständig komplementäre Nukleinsäuren hybridisieren, werden als stringente Hybridisierungsbedingungen bezeichnet. Stringente Hybridisierungsbedingungen sind dem Fachmann bekannt (z.B. Sambrook et al., 1085, Molecular Cloning - A Laboratory Manual, Cold Spring Harbor Laboratory, Cold Spring Harbor, New York). Im allgemeinen, werden stringente Bedingungen so ausgewählt, daß die Schmelztemperatur 5°C niedriger ist als die Tₘ für die spezifische Sequenz bei einer definierten Ionenstärke und pH. Wenn die Hybridisierung unter weniger stringenten Bedingungen durchgeführt wird, dann werden Sequenz-Fehlpaarungen toleriert. Das Ausmaß an Sequenz-Fehlpaarungen kann durch Veränderung der Hybridisierungsbedingungen kontrolliert werden.

Die Durchführung des Hybridisierungsverfahrens ist dem Fachmann an sich bekannt. So werden üblicherweise die festen Phasen nach Inkubation mit der Lösung, die den Hybridisierungspartner enthalten kann, stringenten Bedingungen ausgesetzt, um unspezifisch gebundene Nukleinsäuremoleküle zu entfernen. Die Hybridisierung kann in herkömmlicher Weise auf einer Nylon- oder Nitrocellulosemembran durchgeführt werden (Sambrook et al., Molecular Cloning, Cold Spring Harbor Laboratory, 1989). Die darin genannten Prinzipien lassen sich vom Fachmann auf weitere Ausführungsformen übertragen.

Die Durchführung der Hybridisierung unter stringenten Bedingungen ist besonders wichtig für das erfindungsgemäße Verfahren. Stringent im Sinne der vorliegenden Erfindung bedeutet, daß das Detektionsverfahren eine eindeutige Unterscheidung zwischen einer positiven Reaktion und einer negativen Reaktion bei der Detektion zuläßt. Die Stringenz der Hybridisierung kann durch folgende Maßnahmen verbessert werden:
Struktur der Sonde: Durch die Länge der zur Zielsequenz komplementären Struktur der Sonde; bevorzugt sind 15 bis 20mere.
Laufpuffer: Durch den Salzgehalt wird die Stringenz beeinflußt. Die Ionenstärke liegt bevorzugt zwischen 100-500 mM, insbesondere bevorzugt bei 250 mM.

Weiterhin kann durch mild denaturierende Substanzen im Laufpuffer (DMSO, Formamid, Harnstoff) die Stringenz individuell eingestellt und optimiert werden. Die Stringenz wird auch durch den pH-Wert des Laufpuffers beeinflußt.

Auch die Länge der Zielnukleinsäure spielt eine wichtige Rolle für die Sensitivität der Hybridisierung. Bevorzugt sind Nukleinsäurenstränge mit einer Länge von 100 - 500 Basenpaaren.

Bevorzugt muß die Doppelstrang-Zielnukleinsäure vor der Hybridisierung denaturiert werden. Dies geschieht in der Regel durch basische Chemikalien oder Erwärmung, wobei die für die Doppelstrangstruktur verantwortlichen Wasserstoffbrückenbindungen aufgeschmolzen werden. Bevorzugt als basische Chemikalie ist NaOH in einer Konzentration von 0,1 bis 0,5 M. Besonders bevorzugt ist eine Konzentration von 0,25 M NaOH. Durch Erhitzen einer wäßrigen Nukleinsäurelösung auf mindestens 95°C und anschließendem raschen Abkühlen auf 4°C können ebenfalls Einzelstrangstrukturen erreicht werden. Einzelstrangamplifikate z. B. als Produkte der NASBA-Reaktion sollten vor der Hybridisierung ebenfalls denaturiert werden, um intramolekulare Strukturen aufzulösen. Dies kann wegen der Empfindlichkeit der RNS gegenüber hohen pH-Werten bevorzugt auch durch mild denaturierende Chemikalien wie z.B. DMSO oder Formamid erfolgen.

Die gewünschte Stringenz der Hybridisierung wird neben der Struktur von Zielsequenz und Sonde durch die Zusammensetzung von Hybridisierungs- und Stringenzwaschpuffer bestimmt. Als Hybridisierungspuffer werden in der Regel wäßrige Puffer mit einem Salzgehalt zwischen 0,1 und 0,5 M, und einem pH-Wert von 7,5 - 8,0 verwendet. Für eine gute Benetzung der sondentragenden Phase werden Detergenzien verwendet. Bevorzugt ist Natriumlaurylsulfat (SDS) in einer Konzentration von 0,1 - 7%. In der besonders bevorzugten hohen Konzentration von 7% wirkt SDS zudem günstig auf Signal-/Hintergrundverhältnisse, indem unspezifische Bindungen des Enzymkomplexes unterdrückt werden. Bevorzugt wird nach erfolgter Hybridisierung mit einem Stringenzwaschpuffer inkubiert. Dieser destabilisiert durch eine geringere Ionenstärke den Doppelstrang. So werden nicht 100% komplementäre Hybride wieder getrennt. Durch Zusätze von Chemikalien (z.B. Tetramethylammoniumchlorid, Betain), welche die Wasserstoffbrückenbindungen des Hybrids beeinflussen, lassen sich die Bindungsstärke von G/C und A/T Paarungen angleichen, was bei Multiplexsondensysteme vorteilhaft sein kann.

Erfindungsgemäß wird nach der Hybridisierung das Ausmaß der Hybridisierung bestimmt. Das erfolgt üblicherweise dadurch, daß die Menge der Markierung bestimmt wird, die an eine feste Phase gebunden ist, wobei die Markierung entweder an die Sonden oder die nachzuweisende Nukleinsäure gebunden ist. Derartige Nachweisreaktionen und Detektionsverfahren sind dem Fachmann an sich bekannt.

In einer bevorzugten Ausführungsform des Verfahrens ist die sequenzspezifische Nukleinsäuresonde ausgewählt aus den Sequenzen mit den SEQ ID No.: 10 bis 22 beziehungsweise komplementär zu diesen Sequenzen, beziehungsweise ein Fragment davon beziehungsweise komplementär zu diesem Fragment oder enthält eine dieser Sequenzen beziehungsweise die komplementäre Sequenz.

Eine bevorzugte Ausführungsform des erfindungsgemäßen Verfahrens ist dadurch gekennzeichnet, daß die nachzuweisende Nukleinsäure ein Amplifikationsprodukt ist, wobei die Amplifikation mit sequenzspezifischen Amplifikationsprimern durchgeführt wurde.

Amplifikationsprimer sind ausgewählt aus den Sequenzen mit den SEQ ID No.: 1 -9 beziehungsweise komplementär zu diesen Sequenzen ist, beziehungsweise stellen ein Fragment davon dar beziehungsweise sind komplementär zu diesem Fragment ist oder eine dieser Sequenzen beziehungsweise die komplementäre Sequenz enthält.

Die Amplifikationsprimer sollten so ausgewählt sein, daß das Amplifikationsprodukt gute sterische Verhältnisse in Kombination mit der immobilisierten Sonde aufweist. Pallindromstrukturen, die zu intramolekularen Faltungen führen, können durch geeignete Primerauswahl vermieden werden. Im Falle der Markierung mit Hapten beispelsweise ist die räumliche Anordnung des Haptens (z.B. Biotin) im Hybrid Sonde/Zielnukleinsäure wichtig. Das Hapten sollte für den Antikörper-Enzymkomplex gut zugänglich sein.

Eine Ausführungsform des Verfahrens ist dadurch gekennzeichnet, daß die Nukleinsäuresonden immobilisiert sind. In diesem Falle ist es vorteilhaft, wenn die nachzuweisende Nukleinsäure markiert ist.

In einer anderen Form des Verfahrens sind die Nukleinsäuresonden markiert. In diesem Falle ist es vorteilhaft, wenn die nachzuweisende Nukleinsäure immobilisiert ist.

Gegenstand der Erfindung ist daher ein Verfahren zum Nachweis und zur Differenzierung des *Mycobacterium tuberculosis-Komplex* gemäß den Anspruchen 1-6
- bei welchem eine nachzuweisende Nukleinsäure, welche ein Fragment aus dem Genom eines Mycobakteriums beziehungsweise komplementär zu diesem ist, amplifiziert wird, wobei die Amplifikation mit Primern durchgeführt, von denen mindestens einer eine Sequenz aufweist, die im wesentlichen eine Teilsequenz der nachzuweisenden Nukleinsäure darstellt,
- und bei welchem anschließend die amplifizierte nachzuweisende Nukleinsäure detektiert wird.

Diese Ausführungsform der Erfindung wird im folgenden kurz Amplifikationsverfahren genannt. Der Begriff "Amplifikationsverfahren" schließt alle bevorzugten Ausführungsformen ein.

Dem Fachmann ist bewußt, daß ausgehend von der Lehre der vorliegenden Erfindung auch Primer entworfen werden können, die geringfügig von den erfindungsgemäßen Primern abweichen, aber dennoch funktionieren. So sind auch Primer denkbar, die gegenüber den erfindungsgemäßen Primern am 5'-und/oder 3'-Ende Verlängerungen oder Verkürzungen um wenigstens ein, zwei oder drei Nukleotide aufweisen. Insbesondere Verlängerungen oder Verkürzungen am 5'-Ende der Primer können immer noch funktionsfähige Primer liefern, die erfindungsgemäß eingesetzt werden können. Ebenso ist denkbar, daß einzelne oder wenige Nukleotide eines Primers durch andere Nukleotide austauschbar sind, solange die Spezifität der Primer nicht zu stark verändert wird und der Schmelzpunkt der Primer nicht zu stark verändert wird. Dem Fachmann ist klar, daß neben den üblichen Nukleotiden A, G, C, T auch modifizierte Nukleotide wie Inosin usw. zur Anwendung kommen können. Die Lehre der vorliegenden Erfindung ermöglicht solche Modifikationen, ausgehend vom Gegenstand der Ansprüche.

Am meisten bevorzugt ist das erfindungsgemäße Amplifikationsverfahren, wenn mindestens zwei Primer eine Sequenz aufweisen, die im wesentlichen eine Teilsequenz der nachzuweisenden Nukleinsäure darstellt, wobei die Sequenzen dieser Primer ausgewählt sind aus den Sequenzen mit den SEQ ID No.: 1-9 beziehungsweise komplementär zu diesen Sequenzen sind, beziehungsweise ein Fragment davon darstellt beziehungsweise komplementär zu diesem Fragment ist oder eine dieser Sequenzen beziehungsweise die komplementäre Sequenz enthält. Erfindungsgemäß bevorzugt ist, daß die Primer markiert sind.

Die folgenden Ausführungen gelten sowohl für das erfindungsgemäße Hybridisierungsverfahren als auch für das erfindungsgemäße Amplifikationsverfahren als auch für das gekoppelte Amplifikations-/ Hybridisierungsverfahren. Gekoppelte Amplifikations-/ Hybridisierungsverfahren sind besonders bevorzugt. In diesem Fall wird die Amplifikation mit den erfindungsgemäßen sequenzspezifischen Primern durchgeführt und das so erhaltene Amplifikationsprodukt mit den erfindungsgemäßen sequenzspezifischen Sonden detektiert. Gerade für die Identifizierung und Differenzierung des MTB-Komplexes ist ein sog. "Multiplexansatz" von großem Nutzen, da alle Vertreter dieses Komplexes zu den "langsam wachsenden" Mykobakterien gehören und das Differenzierungsergebnis von großer medizinischer Wichtigkeit ist.

Als Nukleinsäureamplifikationsreaktion können verschiedene Reaktionen eingesetzt werden. Bevorzugt wird die Polymerasekettenreaktion (PCR) eingesetzt. Die verschiedenen Ausgestaltungen der PCR-Technik sind dem Fachmann bekannt, siehe z.B. Mullis (1990) Target amplification for DNA analysis by the polymerase chain reaction. Ann Biol Chem (Paris) 48(8), 579-582. Weitere Amplifikationstechniken, die zur Anwendung kommen können, sind "nucleic acid strand-based amplification" (NASBA), "transcriptase mediated amplification" (TMA), "reverse transcriptase polymerase chain reaction" (RT-PCR), "Q-ß replicase amplification" (ß-Q-Replicase) und die "single strand displacement amplification" (SDA). NASBA und andere Transkriptions-basierte Amplifikationsmethoden werden in Chan und Fox, Reviews in Medical Microbiology (1999), 10 (4), 185-196 erläutert. Im Sinne der vorliegenden Erfindung können jedoch auch nukleinsäuremodifizierende Techniken eingesetzt werden, wie z.B. die reverse Transcription. Letzteres kann eine Modifikation der erfindungsgemäßen Primer notwendig machen, solche Modifikationen sind dem Fachmann bekannt.

In der einfachsten Form der Detektion der nachzuweisenden Nukleinsäure wird das Amplifikat z.B. durch Verdau mit einem Restriktionsenzym spezifisch geschnitten und die entstandenen ethidiumbromidgefärbten Fragmente auf einem Agarosegel analysiert. Weit verbreitet sind auch Hybridisierungsysteme. Die Hybridisierung findet üblicherweise so statt, daß entweder die Zusammensetzung, die das Amplifikationsprodukt oder einen Teil davon enthält, oder die Sonde auf einer festen Phase immobilisiert wird und mit dem jeweils anderen Hybridisierungspartner in Kontakt gebracht wird. Als feste Phasen sind verschiedenste Materialien vorstellbar, beispielsweise Nylon, Nitrocellulose, Polystyrol, silikatische Materialien usw. Es ist auch denkbar, daß als feste Phase eine Mikrotiterplatte eingesetzt wird. Die Zielsequenz kann dabei auch in Lösung zuvor mit einer Fangsonde hybridisieren und danach wird die Fangsonde an eine feste Phase gebunden.

In der Regel ist wenigstens eine Sonde oder wenigstens ein Primer bei der Amplifikation der nachzuweisenden Nukleinsäure markiert.

In einer Ausführungsform des erfindungsgemäßen Verfahrens werden die Nukleinsäuresonden auf der festen Phase immobilisiert, und anschließend wird diese feste Phase mit der Zusammensetzung, welche die nachzuweisenden markierten Nukleinsäuren oder einen Teil davon enthält, in Kontakt gebracht. Vorzugsweise werden wenigstens zwei Sonden auf der festen Phase immobilisiert, bevorzugter wenigstens fünf Sonden, noch bevorzugter wenigstens zehn Sonden. Verschiedene Sonden können in verschiedenen Zonen immobilisiert sein. Durch Inkubation des Amplifikationsprodukts beziehungsweise der Probe enthaltend die nachzuweisende Nukleinsäure oder eines Teils davon mit einer derart vorbereiteten festen Phase mit immobilisierten. Sonden kann durch einen einzigen Hybridisierungsschritt eine Aussage über die Hybridisierung des Amplifikationsprodukts mit allen immobilisierten Sonden gewonnen werden. Die feste Phase ist daher bevorzugt ein Mikroarray von immobilisierten Sonden auf einer festen Phase. Derartige "DNA-Chips" erlauben es, daß auf einem kleinen Bereich eine hohe Anzahl verschiedener Oligonukleotide immobilisiert werden. Die festen Phasen, die für DNA-Chips geeignet sind, bestehen vorzugsweise aus silikatischen Materialien wie Glas usw. Die Markierung der Primer ist in dieser Ausführungsform vorzugsweise eine Fluoreszenzmarkierung. Der DNA-Chip kann nach Inkubation mit dem Amplifikationsprodukt beziehungsweise der Probe enthaltend die nachzuweisende Nukleinsäure oder eines Teils davon durch eine Scanvorrichtung rasch analysiert werden. Derartige Vorrichtungen sind dem Fachmann bekannt. Eine Übersicht über die Chip-Technologie gibt McGlennen (2001) Miniaturization technologies for molecular diagnostics. Clin Chem 47(3), 393-402.

In dieser Ausführungsform des erfindungsgemäßen Verfahrens (im Falle immobilisierter Sonden) ist die nachzuweisende Nukleinsäure markiert. Verschiedenste Markierungen sind dabei denkbar, wie z.B. Fluoreszenzfarbstoffe, Biotin oder Digoxigenin.

Bekannte Fluoreszenzmarkierungen sind Fluoreszein, FITC, Cyaninfarbstoffe, Rhodamine, Rhodamin₆₀₀R-phycoerythrin, Texas Red usw.

Vorstellbar ist auch eine radioaktive Markierung, wie z.B. ¹²⁵I, ³⁵S, ³²P, ³⁵P, oder doppelstrangspezifische Antikörper-Konjugate.

Vorstellbar ist auch eine Partikelmarkierung wie z.B. mit Latex. Solche Partikel sind üblicherweise trocken, im Micron-Bereich und uniform.

Die Markierungen sind üblicherweise kovalent mit den Oligonukleotiden verbunden. Während eine Fluoreszenzmarkierung beispielsweise direkt nachgewiesen werden kann, können Biotin- und Digoxigeninmarkierungen nach Inkubation mit geeigneten Bindemolekülen oder Konjugatspartnern nachgewiesen werden. Andere Bindungspartner als beispielsweise Biotin/Streptavidin sind Antigen/Antibody-Systeme, Hapten/Anti-Hapten-Systeme, Biotin/Avidin, Folsäure/Folat-bindende Proteine, Komplementäre Nukleinsäuren, Proteine A, G und Immunoglobulin usw. (M. N. Bobrov, et al. J. Immunol. Methods, 125, 279, (1989).

Beispielsweise kann ein Biotin-markiertes Oligonukleotid nachgewiesen werden, indem es mit einer Lösung in Kontakt gebracht wird, die Streptavidin gekoppelt an ein Enzym enthält, wobei das Enzym, z.B. Peroxidase oder alkalische Phosphatase, ein Substrat umsetzt, das einen Farbstoff erzeugt oder zu Chemielumineszenz führt. Mögliche Enzyme für diesen Verwendungszweck sind Hydrolasen, Lyasen, Oxido-Reduktasen, Transferasen, Isomerasen und Ligasen. Weitere Beispiele sind Peroxidasen, Glukoseoxidasen, Phosphatasen, Esterasen, und Glykosidasen. Derartige Verfahren sind dem Fachmann an sich bekannt (Wetmur JG. Crit Rev Biochem Mol Biol 1991 ; (3-4) : 227-59; Temsamani J. et al. Mol Biotechnol 1996 Jun;5(3):223-32). Bei manchen Methoden, bei denen Enzyme als Konjugatspartner fungieren, müssen farbändernde Substanzen anwesend sein (Tijssen, P. Practice and Theory of Enzyme Immunoassays in Laboratory Techniques in Biochemistry and Molecular Biology, eds. R. H. Burton and P.H. van Knippenberg (1998).

Ein weiteres bevorzugtes Konjugat umfaßt ein Enzym, welches an einen Antikörper gekoppelt wird (Williams, J. Immunol. Methods, 79, 261 (1984). Weiterhin ist üblich, die Markierung der nachzuweisenden Nukleinsäure mit einem Gold Streptavidin Konjugat durchzuführen, wobei dann ein Biotin-markiertes Oligonukleotid nachgewiesen werden kann. Vorstellbar sind jedoch auch Bindungspartner, die kovalente Bindungen miteinander eingehen, wie z.B. Sulfhydryl-reaktive Gruppen wie Maleimide und Haloacetyl-Derivate und Amin-reaktive Gruppen wie Isothiocyanate, Succinimidylester und Sulfonylhalide.

Werden die nachzuweisenden Nukleinsäuren markiert, dann sind die Sonden in der Regel nicht markiert. Die Markierung der nachzuweisenden Nukleinsäuren erfolgt im wesentlichen nach im Stand der Technik beschriebenen Methoden (US 6,037,127).
Das Einbringen der Markierung in die nachzuweisende Nukleinsäure kann durch chemische oder enzymatische Methoden erfolgen, oder durch direkte Inkorporation von markierten Basen in die nachzuweisende Nukleinsäure. In einer bevorzugten Ausführungsform werden nachzuweisende Sequenzen, die Markierungen inkorporiert haben, durch markierte Basen oder markierte Primer während der Amplifikation der nachzuweisenden Nukleinsäure hergestellt. Markierte Primer können hergestellt werden durch chemische Synthese z.B. mittels der Phosphoramidit-Methode durch die Substitution von Basen des Primers durch markierte Phosphoramiditbasen während der Primer-Synthese. Alternativ dazu können Primer hergestellt werden mit modifizierten Basen, an welche nach der Primer-Synthese Markierungen chemisch gebunden werden.

Denkbar sind auch Verfahren zur Markierung der nachzuweisenden Nukleinsäure ohne daß die zu detektierende Nukleinsäure amplifiziert und/oder mit einer Modifikation versehen wird. Beispielsweise können ribosomale RNS Spezies mit einer DNS-Sonde spezifisch hybridisieren und mit einem RNS/DNS-spezifischen Antikörper als RNS/DNS-Hybrid nachgewiesen werden.

Eine andere Möglichkeit ist das Einbringen von Markierungen mit Hilfe der T4 Polynucleotide-Kinase oder eines terminalen Transferase-Enzyms. Vorstellbar sind so das Einbringen von radioaktiven oder fluoreszierenden Markierungen (Sambrook et. al, Molecular Cloning, Cold Spring Harbor Laboratory Press, Vol. 2, 9.34-9.37 (1989); Cardullo et. al. PNAS, 85, 8790; Morrison, Anal. Biochem, 174, 101 (1988).

Markierungen können in eines oder in beide Enden der Nukleinsäuresequenz der nachzuweisenden Nukleinsäure eingebracht werden. Markierungen können auch innerhalb der Nukleinsäuresequenz der nachzuweisenden Nukleinsäure eingebracht werden. In eine nachzuweisende Nukleinsäure können auch mehrere Markierungen eingebracht werden.

In einer anderen Ausführungsform (in welcher die nachzuweisenden Nukleinsäuren immobilisiert sind) weist wenigstens eine der Sonden eine Markierung auf. Die Markierung der Sonden erfolgt nach den gleichen im Stand der Technik beschriebenen Verfahren wie schon oben für die Markierung der nachzuweisenden Nukleinsäure ausgeführt. Üblicherweise wird dann die Zusammensetzung, die das Amplifikationsprodukt oder einen Teil davon enthält, auf einer festen Phase immobilisiert und mit einer Zusammensetzung in Kontakt gebracht, die wenigstens eine Sonde enthält. Auch in dieser Ausführungsform ist es bevorzugt, eine Hybridisierung mit mehr als einer Sonde durchzuführen. Dazu können mehrere feste Phasen bereitgestellt werden, auf denen das Amplifikationsprodukt beziehungsweise die Probe enthaltend die nachzuweisende Nukleinsäure immobilisiert ist. Es ist aber auch möglich, auf einer festen Phase an mehreren räumlich voneinander getrennten Bereichen kleine Mengen des Amplifikationsprodukts zu immobilisieren. Diese verschiedenen Spots werden dann mit jeweils verschiedenen Sonden in Kontakt gebracht (Hybridisierung).

Gegenstand der vorliegenden Erfindung ist des weiteren eine Vorrichtung zum Nachweis und zur Differenzierung des *Mycobacterium tuberculosis-Komplex* umfassend eine feste Phase, auf der eine oder mehrere sequenz- und/oder speziesspezifische Nukleinsäuresonden immobilisiert sind, gemäß Anspruch 7.

Sequenzspezifische Nukleinsäuresonden sind ausgwählt aus den Sequenzen mit den SEQ ID No.: 10-20 beziehungsweise komplementär zu diesen Sequenzen ist, beziehungsweise ein Fragment davon darstellt beziehungsweise komplementär zu diesem Fragment ist oder eine dieser Sequenzen beziehungsweise die komplementäre Sequenz enthält. Wenn mehrere Oligonukleotide immobilisiert sind, sind diese auf der festen Phase räumlich voneinander getrennt. Vorzugsweise ist die feste Phase als DNA-Chip ausgebildet.

Die feste Phase der erfindungsgemäßen Vorrichtung kann ein chromatographisches Material sein. Da der Analyt hauptsächlich hydrophiler Natur ist, sind hydrophile Eigenschaften des chromatographischen Materials des Teststreifens wichtig für die Durchführung des erfindungsgemäßen Verfahrens. Das chromatographische Material kann umfassen anorganische Puder wie silikatische Materialien, Magnesiumsulfat und Aluminium, kann weiterhin umfassen synthetische oder modifizierte natürlich vorkommende Polymere wie Nitrocellulose, Zelluloseacetat, Zellulose, Polyvinylchlorid oder -acetat, Polyacrylamid, Nylon, vernetztes Dextran, Agarose, Polyacrylat u.s.w., kann weiterhin umfassen beschichtete Werkstoffe wie keramische Materialien und Glas. Am meisten bevorzugt ist die Verwendung von Nitrocellulose als chromatographisches Material. Zusätzlich kann die Einführung von positiv geladenen Ionengruppen in z.B. Nitrocellulose oder Nylonmembranen die hydrophilen Eigenschaften des chromatographischen Materials verbessern.

Das chromatographische Material kann in einem Gehäuse oder ähnlichem montiert sein. Dieses Gehäuse ist in der Regel Wasser unlöslich, rigid und kann aus einer Vielzahl von organischen und anorganischen Materialien bestehen. Wichtig ist, daß das Gehäuse nicht mit den kapillaren Eigenschaften des chromatographischen Materials interferiert, daß das Gehäuse Testkomponenten nicht unspezifisch bindet, und daß das Gehäuse nicht mit dem Detektionssystem interferiert.

Die erfmdungsgemäße Vorrichtung ist bevorzugt, wenn die sequenz- und/oder speziesspezifische Nukleinsäuresonden über einen Linker an die feste Phase der Vorrichtung gebunden ist. Der Linker fungiert als Abstandshalter der Sonde zur Membran. Dies sind im vorliegenden Falle meist Polymere, die den zur Zielsequenz komplementären Teil der Sonde am 5'- oder 3'-Ende verlängern, aber selbst nicht kodierend sind. Dies können Basenabfolgen einer nichtkodierender Nukleinsäuresäurestruktur sein oder andere Polymereinheiten wie z.B. Polyether, Polyester u.ä. Der Linker muß so beschaffen sein, daß er die Hybridisierungseigenschaften der Sonde nicht oder nur schwach negativ beeinflußt wird. Dies kann dadurch vermieden werden, daß keine selbstkomplementären Strukturen vorhanden sind. Auch müssen die chemischen Voraussetzungen für die irreversible Kopplung der Sonde an das Trägermaterial gegeben sein. Eine entscheidende Voraussetzung für ein gutes Funktionieren der Sonde über ihre Eigenschaften ein stabiles Hybrid mit der Zielsequenz zu bilden hinaus, ist die Chemie der Kopplung an die Oberfläche. Es müssen chemische Gruppen vorhanden sein, die bei den verwendeten Immobilisierungstechniken eine irreversible Bindung ermöglichen. Dies können Amine-, Thiolgruppen, Carboimide, Succinimide u.ä. sein.

Dem Fachmann sind jedoch auch andere Möglichkeiten bekannt, Abstandshalter beziehungsweise Linker zwischen der Sonde und der Membran zu schaffen.

Sondenoligonukleotide können beispielsweise über Proteine an die Membranoberfläche gebunden werden. Die mit der Sonde beladenen Proteine können dann nach Standardverfahren an die poröse Membran gebunden werden. Standardverfahren sind zum Beispiel die Kopplung über homobifunktionelle Kopplungsreagenzien oder heterobifunktionelle Kopplungsreagenzien. Bei homobifunktionellen sind die reaktiven Gruppen gleich. Typischerweise sind dies Amine und/oder Thiole. Thiole können synthetisch direkt an Oligonukleotide gekoppelt werden und unter oxidativen Bedingungen mit z.B. Cysteinresten zu Disulfidbrücken reagieren. Für die Kopplung Amin-Amin können Amine als homobifunktionelle Kopplungsreagenzien direkt synthetisch an Oligonukleotide gekoppelt werden und über Imidoester oder Succinimidester an die Oberfläche oder das Protein gebunden werden. Bei heterobifunktionellen Kopplungsreagenzien sind die reaktiven Gruppen unterschiedlich und erlauben die Kopplung von verschiedenen funktionellen Gruppen. Bevorzugt ist die Ausbildung von Amino-Thiol-Kopplungen. Mit einem heterobifunktionellen Kopplungsreagenz, welches sowohl eine Succinimidester-Maleimid oder Iodacetimid beinhaltet, können thiolierte Oligonukleotide gekoppelt werden. Ein weiteres wichtiges Kopplungsagenz sind die Carbodiimide, die Carbonylreste an Amine koppeln. Wichtigster Vertreter ist hier das 1-Ethyl-3-(3-dimethylaminopropyl)carbodiimid (EDAC). Hier können an Membranen mit Carbonylresten aminomodifizierte Oligonukleotide gekoppelt werden. Bei dieser Chemie wird das Kopplungsreagenz nicht in die Verbindung eingebaut.

Verwendung finden im Rahmen der vorliegenden Offenbarung Nukleinsäuren welche ausgewählt sind aus den Sequenzen mit den SEQ ID No.: 1 -22 beziehungsweise komplementär zu diesen Sequenzen ist, beziehungsweise ein Fragment davon darstellt, beziehungsweise komplementär zu diesem Fragment ist oder eine dieser Sequenzen beziehungsweise die komplementäre Sequenz enthält. Gegenstand der vorliegenden Erfindung ist des weiteren eine Zusammensetzung beziehungsweise ein Kit zum Nachweis von Bakterien des *Mycobacterium tuberculosis-Komplex* enthaltend eine oder mehrere der erfindungsgemäßen Nukleinsäuren.

Insbesondere ist Gegenstand der vorliegenden Erfindung ein Kit zur Amplifikation einer nachzuweisenden Nukleinsäure, welche ein Fragment aus dem Genom des *Mycobacterium tuberculosis-Komplex* beziehungsweise komplementär zu diesem ist, enthaltend eine oder mehrere der erfindungsgemäßen Nukleinsäuren.

Zusätzlich zu den erfindungsgemäßen Nukleinsäuren enthält der Kit alle für die Amplifikation der Zielsequenz notwendigen Komponenten, wie Primer, Puffersysteme, Enzyme.

Am meisten bevorzugt ist ein Kit zur Amplifikation einer nachzuweisenden Nukleinsäure, welche ein Fragment aus dem Genom eines *Mycobacterium tuberculosis-Komplex* beziehungsweise komplementär zu diesem ist, enthaltend eine oder mehrere Nukleinsäuren ausgewählt aus den Sequenzen mit den SEQ ID No.: 1-9 beziehungsweise komplementär zu diesen Sequenzen, beziehungsweise ein Fragment davon, beziehungsweise komplementär zu diesem Fragment ist oder eine dieser Sequenzen beziehungsweise die komplementäre Sequenz enthält.

Die Erfindung betrifft auch die Verwendung der erfindungsgemäßen Vorrichtung zum Nachweis und zur Differenzierung des *Mycobacterium tuberculosis-Komplex.*

Die Erfindung betrifft weiterhin die Verwendung der erfindungsgemäßen Nukleinsäure beziehungsweise des erfindungsgemäßen Kits Nachweis und zur Differenzierung des *Mycobacterium tuberculosis-Komplex.*

Die Nukleinsäureamplifikationsreaktion oder -modifikation wird mit einer Probenzusammensetzung durchgeführt. Die Probenzusammensetzung kann jede Zusammensetzung sein, die im Verdacht steht, Bakterien zu enthalten, insbesondere Mykobakterien. Es kann sich um Primärmaterial, z.B. Trachealsekrete, Wundabstriche, Blut usw. handeln oder um Kulturen von Mikroorganismen, die bereits in Flüssig- oder auf Festmedien angezogen wurden. Mit den hier beschriebenen Methoden können die entsprechenden Tuberkulose auslösende Bakterien entweder aus Primärmaterial (z.B. Trachealsekrete, Wundabstriche, Blut u.ä.) oder aus bakteriellen Flüssig- oder Festmedien identifiziert und differenziert werden. Bei nicht primär sterilem Material muß zuvor die nicht säurefeste Begleitflora durch übliche Dekontaminationsverfahren entfernt werden. Dies gilt sowohl für die Anlage der Kultur, als auch für die Vorbehandlung des Primärmaterials.

Das nachfolgende Beispiel soll die Erfindung in Zusammenhang mit den beigefügten Figuren und dem Sequenzprotokoll näher erläutern. Es zeigt:

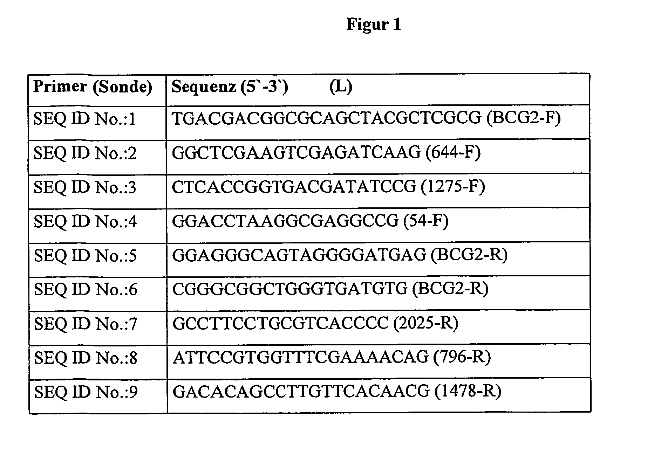

Figur 1: SEQ ID Nos.: 1-9;
Figur 2: SEQ ID Nos.: 10-22

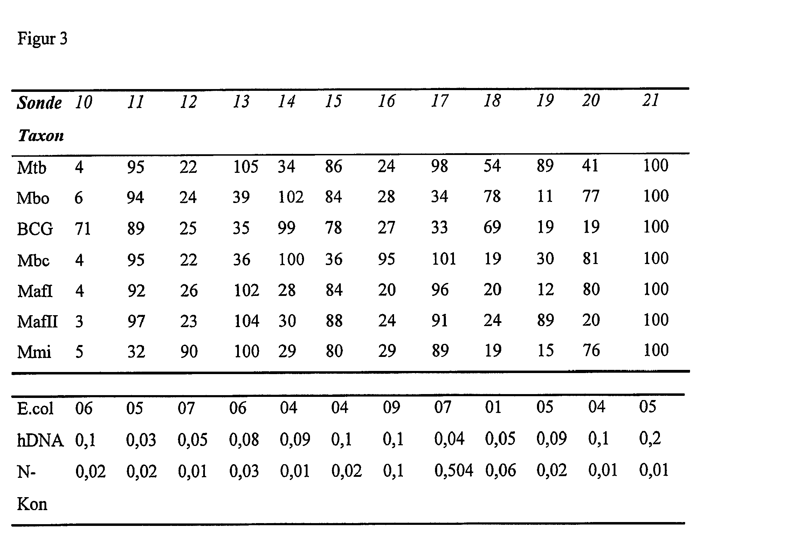

Figur 3: Eine densitometrische Auswertung einer Dot-blot-Hybridisierung zur Bestimmung der Spezifität der Sonden SEQ ID No: 10-21.

Die Abkürzungen haben folgende Bedeutungen:
*Mycobacterium tuberculosis* (ATCC 25177) Mtb,
*Mycobacterium bovis* (ATCC 19210) Mbo,
*Mycobacterium bovis* BCG,
*Mycobacterium bovis* ssp. *caprae* Mbc,
*Mycobacterium africanum* Subtyp I MafI,
*Mycobacterium africanum* Subtyp II MafII,
*Mycobacterium microti* Mmi;
*Escherichia coli* E.col,
humane DNS hDNA,
Negativkontrolle N-Kon;

Die Amplifikation wurde mit den Primern 1 - 9 durchgeführt. Alle Werte sind in % angegeben.Als 100%-Wert wurde der densitometrische Wert der Sonde 21 bestimmt. Dadurch können sich im Einzelfall Werte > 100% ergeben.

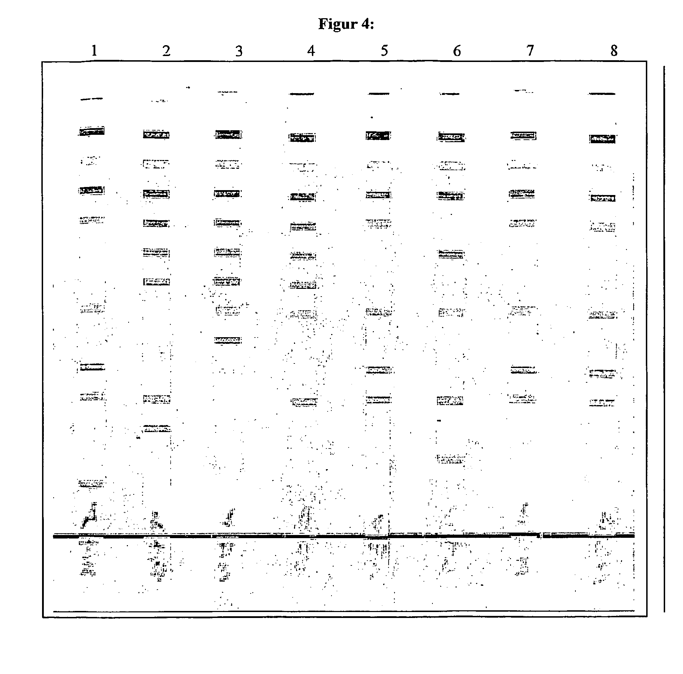

Figur 4: Einen reversen Hybridisierungsstreifen mit der Probenreihenfolge:
1.BCG
2.M. microti
3.M. tuberculosis
4.M. africanum I
5.M. bovis ssp. bovis
6.M. bovis ssp. caprae
7.M. bovis ssp. bovis
8.M. bovis ssp. bovis

Auftragsreihenfolge Sonden auf Membran MC120301A

| **Linie Nr.** | **Sondenbezeichnung** |
|---|---|
| 1 | Biotinylierte DNS |
| 2 | Seq ID No. 22 |
| 3 | Seq ID No. 21 |
| 4 | Seq ID No. 15 |
| 5 | Seq ID No. 17 |
| 6 | Seq ID No. 13 |
| 7 | Seq ID No. 11 |
| 8 | Seq ID No. 19 |
| 9 | Seq ID No. 18 |
| 10 | Seq ID No. 20 |
| 11 | Seq ID No. 12 |
| 12 | Seq ID No. 16 |
| 13 | Seq ID No. 10 |

Die Sonde Seq ID No. 22 erfasst alle Spezies der Gattung Mykobakterien. Sie kontrolliert die erfolgreiche Amplifikation und dient als Referenz zur Abgleichung der Bandenstärke und der Interpretation der Banden. Die Amplifikation erfolgte mit der Methode der PCR mit biotinylierten Primern Seq ID No. 1 - 9.

SEQ ID Nos 1 - 22: Erfindungsgemäß verwendete Sonden zur Amplifikation und/oder dem Nachweis von MBTK

### Beispiel

### DNS/RNS-Isolierung:

Bakterielle Nukleinsäure wurde entweder von Festnährmedien, Flüssigmedien oder aus Primärmaterial nach entsprechender Vorbehandlung gewonnen.

**Tabelle 1: Bakterienspezies, die in Beispiel 1 untersucht wurden**

| **Spezies** | **Abkürzung** | **ATCC Nr.** |
|---|---|---|
| Mycobacterium tuberulosis | Mtb | 25177 |
| Mycobacterium bovis ssp. bovis | Mbo | 19210 |
| Mycobacterium bovis BCG | BCG | / |
| Mycobacterium bovis ssp. caprae | Mbc | / |
| Mycobacterium africanum Subtyp I | MafI | / |
| Mycobacterium africanum Subtyp II | MafII | / |
| Mycobacterium microti | Mmi | / |

Dazu wurde von Festmedien mit einer sterilen Impföse bakterielles Material entnommen und in 300µl 10mM Tris/HCl pH 7,5 suspendiert. Aus Flüssigkulturen wurde 1ml entnommen, 5min bei 13.000rpm in einer Tischzentrifuge zentrifugiert, der Überstand verworfen und in 300µl 10mM Tris/HCl pH 7,5 resuspendiert. Primärmaterial wurde mit Acetylcystein verflüssigt und mit NaOH/SDS "dekontaminiert". Die so erhaltenen Zellsuspensionen wurden 15min bei 95°C in einem Thermomixer (Eppendorf, Hamburg, Deutschland) inkubiert, 15min in einem Ultraschallbad (Bandelin) beschallt und 10min bei 13.000rpm in einer Tischzentrifuge zentrifugiert. Vom Überstand wurden jeweils 5µl in die Amplifikationsreaktion eingesetzt.

### Amplifikation:

Alle Primer und Sonden (SEQ ID.No. 1-22) wurden kommerziell synthetisiert (Interactiva, Ulm, Deutschland). Der PCR-Ansatz enthielt 1 x Taq-Puffer (Qiagen, Hilden, Deutschland), je 1µM Primer, 200µM dNTP (Roche) und 1U Hotstar Taq-Polymerase (Qiagen, Hilden, Deutschland). Die PCR-Amplifikation wurde auf einem Thermocycler PE 9600 (ABI, Weiterstadt, Deutschland) mit 15min 95°C, 10 Zyklen mit 30sek 95°C und 2min 60°C und mit 20 Zyklen 10sek 95°C, 50sek 55°C und 30sek 70°C durchgeführt.

Für die RNA-Amplifikation mit der NASBA-Technik wurde das NucliSens Amplifikationskit (Organon Technika, Boxtel, Niederlande) nach Angaben des Herstellers verwendet:
1. Herstellung des Amplifikationsmixes: 8"1 "reagent sph"re" "n "reagent dilut"on"-Puffer gelöst (enthält die für die Reaktion benötigten Enzyme), 5µl KCl-Lösung, Endkonzentration 70mM KCl und 2µl Primerlösung, Endkonzentration 0,5µM Primer;
2. 5µl RNA-Lösung zugeben und 60min bei 41°C im Wasserbad inkubieren.

DNA/RNA-Amplifikat wurde entweder mit einem ethidiumbromid-gefärbtem Agarosegel oder durch Hybridisierung nachgewiesen.

### Amplifikation:

Der PCR-Ansatz enthielt 1 x Taq-Puffer (Qiagen, Hilden, Deutschland), je 1µM Primer SEQ.IDNo.-1 - 9, 200µM dNTP (Roche) und 1U Hotstar Taq-Polymerase (Qiagen, Hilden, Deutschland). Die PCR-Amplifikation wurde auf einem Thermocycler PE 9600 (ABI, Weiterstadt, Deutschland) mit 15min 95°C, 10 Zyklen mit 30sek 95°C und 2min 60°C und mit 20 Zyklen 10sek 95°C, 50sek 55°C und 30sek 70°C durchgeführt.

### Herstellung des Hybridisierungsstreifens mit immobilisierten Sonden:

Eine Nylonmembran (Biodyne A, Pall, Portsmouth, England) wurden auf die Größe der Blotapparatur geschnitten und mit 10 x SSC getränkt. In die Öffnungen der zusammengebauten Apparatur wurden 250µl einer 1 µM Sondenlösung der SEQ.ID Nos. 10-22 und 15-22 in 10 x SSC in schlitzförmigen Öffnungen der Blotapparatur vorgelegt. Nach Anlegen eines Vakuums wurde gewartet, bis alle Flüssigkeit vollständig durchgesaugt war. Anschließend wurde mit 10 x SSC-Puffer nachgespült. Die Membran wurde, nachdem sie vollständig getrocknet war in einem UV-Crosslinker (UV-Stratalinker 2400, Stratagene, La Jolla, USA) bei 1200 Joule/cm² fixiert und mit destilliertem Wasser gewaschen, getrocknet und in ca.4mm breite Streifen geschnitten.

Alle Hybridisierungen wurden bei 50°C in einem Wasserbad (Thermolab, GFL, Burgwedel, Deutwschland) durchgeführt. Die mit Sonden beschichtete Membran wurde in Streifen geschnitten und in einer Inkubationswanne (Coming costar, Bodenheim, Deutschland) mit vorgewärmtem Hybridisierungspuffer inkubiert. Nach Zugabe von 20µl in 0,4M NaOH denaturiertem Amplifikat erfolgte für eine Stunde die Hybridisierungsreaktion. Nicht gebundenes oder nur partiell gebundenes Amplifikat wurde durch 30min Inkubation mit Stringent-Puffer bei 50°C mit einmaligem Tausch des vorgewärmten Stringent-Puffers entfernt. Anschließend wurde Blocking-Reagens zugegeben und 15min bei 37°C inkubiert. Die Hybride wurden kolorimetrisch durch ein Streptavidin-Alkalische Phosphatase-Konjugat mit Zugabe von NBT/BCIP detektiert. Dazu wurde Streptavidin-Alkalische Phosphatase-Konjugat zugegeben und 30min bei 37°C inkubiert. Anschließend wurde die Membran zweimal 15min mit Substratpuffer gewaschen. Die Membran wurde in Substratpuffer mit NBT/BCIP 10min inkubiert und die Farbentwicklung durch Waschen mit H₂O_{bidest} gestoppt.

### Detektion der Amplifikate durch Sondenhybridisierung:

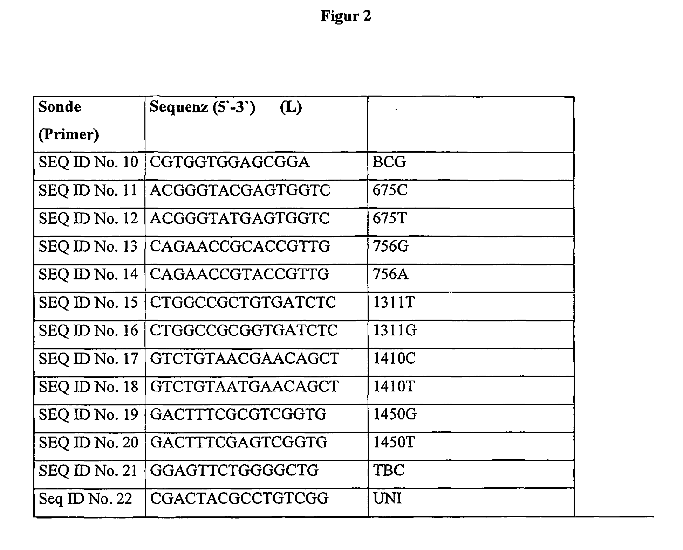

Alle Sonden wurden am 5'-Ende biotiniliert, um Zielsequenz/Sonden-Hybride über an Streptavidin gekoppelte Reporterenzyme nachweisen zu können. Als Sonden werden Oligonukleotide mit den Sequenzen SEQ ID NO. 10 bis 22 eingesetzt (siehe Figur 2A).

Saugfähiges Papier (Blotting Papier GB002, Schleicher & Schüll, Dassel, Deutschland), und eine Nylonmembran (Biodyne A, Pall, Portsmouth, England) wurden auf die Größe der Blotapparatur (Minifold Schleicher & Schüll, Dassel, Deutschland) geschnitten und mit 10 x SSC getränkt. In die Öffnungen der zusammengebauten Apparatur wurden 250µl Denaturierungslösung (50 mM NaOH; 1,5 M NaCl) vorgelegt und 20 µl Amplifikat zupipettiert. Nach Anlegen eines Vakuums wurde gewartet, bis alle Flüssigkeit vollständig durchgesaugt war. Anschließend wurde mit 10 x SSC-Puffer nachgespült. Die Membran wurde, nachdem sie vollständig getrocknet war in einem UV-Crosslinker (UV-Stratalinker 2400, Stratagene, La Jolla, USA) bei 1200 Joule/cm² fixiert und mit destilliertem Wasser gewaschen und getrocknet.

Alle Hybridisierungen wurden bei 50°C in einem Hybridisierungsofen (Hybaid Mini Oven MkII, MWG-Biotech, Ebersberg, Deutschland) in Glasröhren durchgeführt. Die dannDNA/RNA-Amplifikat beschichtete Membran wurde in trockenem Zustand eingerollt und in eine Glasröhre gegeben. Anschließend wurde die Membran unter ständiger Rotation 5min mit vorgewärmten Hybridisierungspuffer inkubiert. Nach Zugabe von 2 pmol biotinilierter Sonde erfolgte für eine Stunde die Hybridisierungsreaktion. Nichtgebundene oder nur partiell gebundene Sonde wurde durch 30min Inkubation mit Stringent-Puffer bei 50°C mit einmaligem Tausch des vorgewärmten Stringent-Puffers entfernt. Anschließend wurde Blocking-Reagens zugegeben und 15min bei 37°C weiterinkubiert. Die Hybride wurden durch ein Streptavidin-Alkalische Phosphatase-Konjugat durch Zugabe von NBT/BCIP kolorimetrisch oder durch Aufsprühen von Chemolumineszenzsubstrat (Lumi-Phos 530, Cellmark Diagnostics, Abindon, England) autoradiographisch detektiert. Dazu wurde Streptavidin-Alkalische Phosphatase-Konjugat zugegeben und 30min bei 37°C inkubiert. Anschließend wurde die Membran zweimal 15min mit Substratpuffer gewaschen. Die Membran wurde dann entnommen, Lumi-Phos-Reagens wurde aufgesprüht, gefolgt von 2h Exposition eines Röntgenfilms. Alternativ dazu wurde Substratpuffer mit NBT/BCIP zugegeben und die Farbentwicklung abgewartet.

### Verwendete Lösungen:

10 x SSC-Lösung (Standard-Saline-Citrat):
1,5M NaCl, 0,15M Trinatriumcitrat;

### Hybridisierungspuffer:

7% SDS (Na-dodecylsulfat), 0,25M Phosphatpuffer pH 7,5;

### Stringentwaschlösung (Stringent-Puffer):

3M TMCL (Tetramethylammoniumchlorid), 50mM Tris/Cl, 2mM EDTA, 0, 1 % SDS;

### Lösung zur Absättigung der Membranbindungsstellen:

5g/l Blockingreagenz (Roche) in Maleinsäurepuffer pH 7,5 (4,13g NaCl und 5,53g Maleinsäure in 500ml Wasser, pH mit 5M NaOH auf 7,5 eingestellt);

### Substratpuffer:

274mM Tris/Cl pH 7,5, 68,6 mM Na₃Citrat, 200mM NaCl, 27,4 mM MgCl₂ * 6 H₂O; Lumiphos (Cellmark, Großbritannien)

## Patentansprüche

1. Multiplexamplifikationsverfahren zum Nachweis und zur Differenzierung des *Mycobacterium tuberculosis-Komplex,* bei welchem eine nachzuweisende Nukleinsäure, welche ein Fragment aus dem Genom eines *Mycobacterium tuberculosis-Komplex* ist oder komplementär zu diesem ist, an eine sequenz- und/oder speziesspezifische Nukleinsäuresonde hybridisiert und anschließend die nachzuweisende Nukleinsäure beziehungsweise die Hybridisierung der nachzuweisenden Nukleinsäure an die sequenzspezifische Nukleinsäuresonde detektiert wird, **dadurch gekennzeichnet,**
**daß** die nachzuweisende Nukleinsäure ein Amplifikationsprodukt ist, wobei die Amplifikation mit mindestens zwei sequenzspezifischen Amplifikationsprimern durchgeführt wird und wenigstens ein Amplifikationsprimer ausgewählt ist aus den Sequenzen mit den SEQ ID No.: 1-3, 5, 6, 8, 9 oder hierzu komplementären Sequenzen und
**daß** die sequenzspezifische Nukleinsäuresonde ausgewählt ist aus den Sequenzen mit den SEQ ID No.: 10-20, 22 oder hierzu komplementären Sequenzen.

2. Verfahren gemäß Anspruch 1, **dadurch gekennzeichnet, daß** die Nukleinsäuresonden immobilisiert sind.

3. Verfahren Anspruch 1, **dadurch gekennzeichnet, daß** die nachzuweisende Nukleinsäure markiert ist.

4. Verfahren gemäß einem Anspruch 1-3, **dadurch gekennzeichnet, daß** die Nukleinsäuresonden markiert sind und die nachzuweisende Nukleinsäure immobilisiert ist.

5. Verfahren gemäß einem der Ansprüche 1 bis 4, **dadurch gekennzeichnet, daß** die Amplifikation mit der Polymerase-Ketten-Reaktion erfolgt.

6. Verfahren gemäß einer der Ansprüche 1 bis 5 **dadurch gekennzeichnet, daß** die Primer markiert sind.

7. Vorrichtung zum Nachweis und zur Differenzierung des *Mycobacterium tuberculosis-Komplex* umfassend eine feste Phase, auf der mehrere sequenz- und speziesspezifische Nukleinsäuresonden immobilisiert sind, **dadurch gekennzeichnet, daß** die sequenzspezifische Nukleinsäuresonde ausgewählt ist aus den Sequenzen mit den SEQ ID No.: 10-20 beziehungsweise komplementär zu diesen Sequenzen ist.

8. Vorrichtung gemäß Anspruch 7 **dadurch gekennzeichnet, daß** die sequenz- und speziesspezifische Nukleinsäuresonden über einen Linker an die feste Phase der Vorrichtung gebunden ist.

9. Kit zur Amplifikation einer nachzuweisenden Nukleinsäure, welche ein Fragment aus dem Genom eines *Mycobacterium tuberculosis-Komplex* beziehungsweise komplementär zu diesem ist, enthaltend mehrere Nukleinsäuren als Primer ausgewählt aus den Sequenzen mit den SEQ ID No.: 1-3, 5, 6, 8, 9 oder deren komplementäre Sequenzen.

10. Kit nach Anspruch 9 zum Nachweis und zur Differenzierung des *Mycobacterium tuberculosis-Komplex* enthaltend mehrere Nukleinsäuren ausgewählt aus den Sequenzen mit den SEQ ID No.: 10-20, 22 beziehungsweise komplementär zu diesen Sequenzen.

11. Verwendung der Vorrichtung gemäß einem der Ansprüche 7 bis 8 zum Nachweis und zur Differenzierung des *Mycobacterium tuberculosis-Komplex.*

12. Verwendung des Kits gemäß einem der Ansprüche 9 oder 10 zum Nachweis und zur Differenzierung des *Mycobacterium tuberculosis-Komplex.*

## Claims

1. A multiplex amplification method for detecting and for differentiating the *Mycobacterium tuberculosis complex* in which a nucleic acid to be detected, which is a fragment from the genome of a *Mycobacterium tuberculosis complex* or is complementary to the latter, is hybridized onto a sequence- and/or species-specific nucleic acid probe, and subsequently the nucleic acid to be detected or the hybridization of the nucleic acid to be detected onto the sequence-specific nucleic acid probe is detected, **characterized in that** the nucleic acid to be detected is an amplification product, where the amplification is carried out with at least two sequence-specific amplification primers and at least one amplification primer is selected from the sequences having SEQ ID No.: 1-3, 5, 6, 8, 9 or is complementary to these sequences and **in that** the sequence-specific nucleic acid probe is selected from the sequences having the SEQ ID No.: 10-20, 22 or is complementary to these sequences.

2. The method as claimed in claim 1, **characterized in that** the nucleic acid probes are immobilized.

3. The method of claim 1, **characterized in that** the nucleic acid to be detected is labeled.

4. The method as claimed in any one of claims 1-3, **characterized in that** the nucleic acid probes are labeled, and the nucleic acid to be detected is immobilized.

5. The method as claimed in any one of claims 1 to 4, **characterized in that** the amplification takes place with the polymerase chain reaction.

6. The method as claimed in any one of claims 1 to 5, **characterized in that** the primers are labeled.

7. A device for the detection and differentiation of the *Mycobacterium tuberculosis complex* comprising a solid phase on which several sequence- and species-specific nucleic acid probes are immobilized, **characterized in that** the sequence-specific nucleic acid probe is selected from the sequences having SEQ ID No.:10-20, or is complementary to these sequences.

8. The device as claimed in claim 7, **characterized in that** the sequence- and species-specific nucleic acid probe is linked to the solid phase of the device via a linker.

9. A kit for amplification of a nucleic acid to be detected, which is a fragment from the genome of a *Mycobacterium tuberculosis complex* or is complementary thereto, comprising several nucleic acids as primer selected from the sequences having SEQ ID No.:1-3, 5, 6, 8, 9, or is complementary to these sequences.

10. A kit as claimed in claim 9 for the detection and differentiation of the *Mycobacterium tuberculosis* complex comprising several nucleic acids selected from the sequences having SEQ ID No.:10-20, 22, or is complementary to these sequences.

11. The use of the device as claimed in any one of claims 7 to 8 for the detection and differentiation of the *Mycobacterium tuberculosis complex.*

12. The use of the kit as claimed in any one of claims 9 or 10 for the detection and differentiation of the *Mycobacterium tuberculosis complex.*

## Revendications

1. Procédé d'amplification multiplex pour détecter et différencier le *complexe Mycobacterium tuberculosis,* selon lequel un acide nucléique à détecter, lequel est un fragment du génome d'un *complexe Mycobacterium tuberculosis* ou est complémentaire de celui-ci, est hybridé à une sonde d'acide nucléique spécifique à une séquence et/ou spécifique à une espèce et l'acide nucléique à détecter ou encore l'hybridation de l'acide nucléique à détecter est détecté sur la sonde d'acide nucléique spécifique à une séquence, **caractérisé en ce que** l'acide nucléique à détecter est un produit d'amplification, l'amplification étant effectuée avec au moins deux amorces d'amplification spécifiques à une séquence et au moins une amorce d'amplification étant sélectionnée parmi les séquences ayant les SEQ ID No. : 1-3, 5, 6, 8, 9 ou des séquences complémentaires à celles-ci et **en ce que** la sonde d'acide nucléique spécifique à une séquence est sélectionnée parmi les séquences ayant les SEQ ID NO.: 10-20, 22 ou des séquences complémentaires à celles-ci.

2. Procédé selon la revendication 1, **caractérisé en ce que** les sondes d'acide nucléique sont immobilisées.

3. Procédé selon la revendication 1, **caractérisé en ce que** l'acide nucléique à détecter est marqué.

4. Procédé selon une quelconque revendication 1 - 3, **caractérisé en ce que** les sondes d'acide nucléique sont marquées et l'acide nucléique à détecter est immobilisé.

5. Procédé selon l'une quelconque des revendications 1 à 4, **caractérisé en ce que** l'amplification a lieu par la réaction en chaîne par polymérase.

6. Procédé selon l'une quelconque des revendications 1 à 5, **caractérisé en ce que** les amorces sont marquées.

7. Dispositif de détection et de différenciation du *complexe Mycobacterium tuberculosis* comprenant une phase solide, sur laquelle plusieurs sondes plusieurs sondes d'acide nucléique spécifiques à une séquence et à une espèce sont immobilisées, **caractérisé en ce que** la sonde spécifique à une séquence est sélectionnée parmi les séquences ayant les SEQ ID No. : 10-20 ou est complémentaire à ces séquences.

8. Dispositif selon la revendication 7, **caractérisé en ce que** la sonde d'acide nucléique spécifique à une séquence et à une espèce est liée par un lieur à la phase solide du dispositif.

9. Kit d'amplification d'un acide nucléique à détecter, lequel est un fragment du génome d'un *complexe Mycobacterium tuberculosis* ou est complémentaire de celui-ci, contenant comme amorces plusieurs acides nucléiques sélectionnés parmi les séquences ayant les SEQ ID No. : 1-3, 5, 6, 8, 9 ou leurs séquences complémentaires.

10. Kit selon la revendication 9 pour détecter et différencier le *complexe Mycobacterium tuberculosis* contenant plusieurs acides nucléiques sélectionnés parmi les séquences ayant les SEQ ID No. : 10-20, 22 ou complémentaires à celles-ci.

11. Utilisation du dispositif selon l'une quelconque des revendications 7 à 8 pour détecter et différencier le *complexe Mycobacterium tuberculosis.*

12. Utilisation du kit selon l'une quelconque des revendications 9 ou 10 pour détecter et différencier le *complexe Mycobacterium tuberculosis.*
